# EUROPEAN PATENT APPLICATION

(11) **EP 4 167 245 A1**
(43) Date of publication of application: **19.04.2023**
(21) Application number: 21203074.6
(22) Date of filing: 18.10.2021
(51) Int. Cl.: G16H 50/20, G06N 3/00, G06N 20/00, G16H 50/30, G16H 50/50, G16H 50/70

(54) **SYSTEMS AND METHODS FOR MODELLING A HUMAN SUBJECT**

(71) Applicant: Koninklijke Philips N.V., 5656 AG Eindhoven (NL)
(72) Inventor: CRONIE, Harm, Eindhoven (NL); COX, Lieke Gertruda Elisabeth, Eindhoven (NL); BULUT, Murtaza, Eindhoven (NL); LAVEZZO, Valentina, Eindhoven (NL); HENDRIKS, Cornelis Petrus, Eindhoven (NL)
(74) Representative: Philips Intellectual Property & Standards

(57) **Abstract**

Systems and methods are proposed for training and employing a machine learning algorithm for identifying uncertain Digital Twin model parameters. Such proposals may make use of a set of DTs that is chosen such that it comprises DTs that have achieved a target/predetermined morbidity scenario. The training may then identify uncertain model parameters for the set of DTs. Using the identified uncertain model parameters, the set of DTs may then be run so as to assess whether the target morbidity scenario is achieved.

## Description

### FIELD OF THE INVENTION

The invention relates to modelling human subjects, and more particularly to the field of models of biological function (commonly referred to as digital twins).

### BACKGROUND OF THE INVENTION

A recent development in healthcare is the so-called 'digital twin' concept. In this concept, a digital representation or computational simulation (i.e. the Digital Twin (DT)) of a physical system is provided and connected to its physical counterpart, for example through the Internet of things as explained in US 2017/286572 A1 for example. Through this connection, the DT typically receives data pertaining to the state of the physical system, such as sensor readings or the like, based on which the digital twin can predict the actual or future status of the physical system, e.g. through simulation.

A DT has been defined as "a set of virtual information constructs that mimics the structure, context and behavior of an individual or unique physical asset that is dynamically
updated with data from its physical twin throughout its life-cycle. Although this definition targets engineering systems, a broad interpretation covers the use of DT in a diverse array of other application areas such as healthcare and information systems. Thus, DTs may be constructed for processes and living entities, and the lifecycle may be the period over which the digital twin is needed to support decision-making. DT may also be used over fixed time periods, such as for surgery or in critical decision points for physical systems. In essence, a digital twin is an in-silico model that brings together the technology to map, monitor and control real-world entities by continually receiving and integrating data from a physical twin to provide an up-to-date digital representation of the physical entity.

Such DT technology is also becoming of interest in the medical field, as it provides an approach to more efficient medical care provision. For example, a DT may be built using imaging data of a subject (i.e. patient), e.g. a person suffering from a diagnosed medical condition as captured in the imaging data.

The DT(s) of a subject (i.e. subject-specific computational simulations) may serve a number of purposes. Firstly, the DT(s) (rather than the patient) may be subjected to a number of virtual tests, e.g. treatment plans, to determine which treatment plan is most likely to be successful to the patient. This reduces the number of tests that physically need to be performed on the actual patient. Secondly, the DT(s) of a subject may be used to predict the onset, treatment (outcome) or development of medical conditions of the subject. That is, the DT(s) of a subject may offer healthcare professionals advanced visualization and/or physical insights into health information of the subject, thus supporting improved Clinical Decision Support (CDS).

DTs are therefore powerful and complex tools with capabilities that can directly influence healthcare options and services that a user can/will receive. Moreover, DTs can have a direct influence on lifestyle choices of a user (e.g. when used outside the healthcare context).

The main components of a DT tool are bio-physical and/or data-driven models that represent the physical object or process. Such models can be complex in nature and comprise a high number of model parameters. In many cases, there is uncertainty present on the exact value of such parameters, thus leading to uncertainty in the outcome of a simulation.

For example, in a clinical setting, a digital twin model of a patient may be used to assess the morbidity that results from a set of potential interventions to treat a particular condition. Uncertain model parameters may include intrinsic uncertainties related to the digital model such as renal function, effectiveness of digestive system, and psychological status. An example of an extrinsic uncertainty is, for instance, the exact medication schedule of the patient which in turn leads to e.g. fluctuations of medication concentration in the subject's (i.e. patient's) cardiovascular system. A constraint of a medical setting is there is typically only a limited amount of time available to consider different values for uncertain model parameters in an attempt to cover relevant/potential morbidity scenarios.

### SUMMARY OF THE INVENTION

The invention is defined by the claims.

According to examples in accordance with an aspect of the invention, there is provided a method of training a machine learning algorithm for identifying parameter values for a digital twin of a biological asset of a subject, the method comprising:
selecting, from a plurality of digital twins, a subset of the plurality of digital twins, each digital twin of the subset having one or more uncertain model parameters;
removing, from the subset, digital twins for which a target morbidity scenario has not been previously obtained, so as to provide a training set of digital twins; and
training the machine learning algorithm based on the remaining digital twins of the subset to generate or identify the uncertain model parameters.

Embodiments propose concepts for training an artificial intelligence (AI) model on a population of DTs. Such proposals are based on a realization that such training may identify uncertain model parameters, thus enabling the use of fewer simulations to cover a target/predetermined morbidity. That is, proposed are concepts that make use of a set of DTs to train a machine learning algorithm. By choosing this set of DTs such that it comprises DTs that achieved the target/predetermined morbidity scenario, a generative AI model may generate uncertain model parameters for the (set of) target DT(s). Using the generated model parameters, the (set of) target DT(s) may then be run so as to assess whether the target morbidity scenario is achieved.

Proposed embodiments may thus provide a DT simulation platform where uncertain model parameters are generated.

By way of example, embodiments may propose the training and/or use of a machine learning algorithm which facilitates the simulation of a complication (e.g. in advance) with a reduced number of simulation runs/executions. Such embodiments may further enable a user to assess severity and potentially investigate (e.g. simulate) responses and their outcomes.

Embodiments thus propose one or more concepts for training a generative AI model which makes use of a population of DTs. The generative AI model may be trained on a population of DT models that achieved a target morbidity scenario, so as to identify uncertain model parameters. Uncertain model parameters generated by the AI model may be used for target DT simulations so that the target morbidity scenario is covered with a reduced/minimal runs.

Proposed embodiments may thus provide dynamic DT simulation concepts that cater for changing parameters and/or conditions.

The input of the machine learning algorithm may be anything that relates to the DT model that is not part of the uncertain model parameters, for example geometry, textual description encoded into suitable representation (e.g. embedding), a subset of past simulation results, etc. Thus, training may be based on e.g. the specific geometric data in a digital twin model. An actual representation of the target morbidity scenario may also be included as input data for the training. This may be useful when an end-user is interested in multiple morbidity scenarios at once or when the target morbidity scenario is described by a range of values and for one model the particular morbidity takes just one value from this range.

In some embodiments, the training comprises: defining a value for each of the one or more uncertain model parameters of a first digital twin of the training set; performing a simulation using the first digital twin to obtain a simulation result; and adjusting a configuration of the machine learning algorithm based on the simulation result.

For example, defining a value for each of the one or more uncertain model parameters comprises either: determining a random value; or perturbing an existing parameter value.

By way of further example, the training may comprise determining values for the one or more uncertain model parameters of the first digital twin that provide a simulation result adhering to the target morbidity scenario.

In some embodiments, training may comprise adjusting a configuration of the machine learning algorithm so as to decrease a difference between one or more target parameter values of a first digital twin of the training set for obtaining the target morbidity scenario and one or more predicted parameter values that are output by the machine learning component.

By way of example, the machine learning algorithm may comprise an encoder-type neural network or a generative adversarial neural network. That is, embodiments may employ a generative AI model that may be a generative adversarial neural network or a network that is configured to perform neural importance sampling.

According to another aspect of the invention, there is provided a method for predicting morbidity scenario risk for a subject, the method comprising:
determining parameter values for a digital twin of a biological asset of the subject using a machine learning algorithm trained according to a proposed embodiment; and
executing a plurality of simulations with the digital twin for different input values to the digital twin so as obtain a respective plurality of prediction results, each prediction result comprising an indication of morbidity scenario occurrence.

Proposed embodiments may thus provide a DT simulation platform to assess morbidity, wherein uncertainty is reduced by using historical DT simulations.

Some embodiments may further comprise analyzing the respective plurality of prediction results to identify a digital twin parameter of interest, the digital twin parameter of interest having a level of influence on morbidity scenario occurrence that exceeds a threshold value.

Purely by way of example, analyzing may comprise processing the plurality of prediction results with at least one of: a sampling algorithm and a data clustering algorithm. Such a clustering algorithm may, for instance, be configured to discover different classes of uncertain model parameters for which a morbidity is achieved.

The machine learning algorithm may be trained using a training algorithm configured to receive an array of training inputs and known outputs, wherein the training inputs comprise a representation of a morbidity scenario and the known outputs comprise one or more parameter values for digital twins for predicting the morbidity scenario. The representation of the morbidity scenario may include everything related to the DT model except uncertain model parameters. The training inputs may also include a representation of the morbidity scenario (e.g. text, real number, or some suitable encoding of the morbidity scenario).

Some embodiments may further comprise communicating at least one of the prediction results to computing device based on the morbidity scenario.

Embodiments may, for example, be employed to check which parameter (for an individual subject) is most critical in determining the risk of a certain complication. Furthermore, a threshold for such a parameter may also be determined (i.e. a subject-specific threshold), which may be distinct from a generally used population-based threshold.

Also, by including probability estimation of the parameter leading to one or more target morbidity scenarios, a subject-specific risk analysis may be performed.

According to examples in accordance with yet another aspect of the invention, there is provided a computer program product comprising computer program code means which, when executed on a computing device having a processing system, cause the processing system to perform all of the steps of the methods described above.

According to another aspect of the invention, there is provided a system for training a machine learning algorithm for identifying parameter values for a digital twin of a biological asset of a subject, the system comprising:
a processor arrangement configured to perform the steps of:
selecting, from a plurality of digital twins, a subset of the plurality of digital twins, each digital twin of the subset having one or more uncertain model parameters;
   removing, from the subset, digital twins for which a target morbidity scenario has not been previously obtained, so as to provide a training set of digital twins; and
   training the machine learning algorithm based on the remaining digital twins of the subset excluding their uncertain model parameters.

According to another aspect, there is provided a system for predicting morbidity scenario risk for a subject, the system comprising:
a processor arrangement configured to perform the steps of:
determining parameter values for a digital twin of a biological asset of the subject using a machine learning algorithm trained according to a proposed embodiment; and
executing a plurality of simulations with the digital twin for different input values to the digital twin so as obtain a respective plurality of prediction results, each prediction result comprising an indication of morbidity scenario occurrence.

Further, proposed concepts may provide a clinical decision support comprising a system according to a proposed embodiment. Also, embodiments may be employed in combination with conventional/existing clinical decision support systems. In this way, embodiments may integrate into legacy systems so as to improve and/or extend their functionality and capabilities. An improved clinical decision support system may therefore be provided by proposed embodiments.

Embodiments may therefore be of particular use in relation to DTs that support clinical decision making. Exemplary usage applications may, for example, include the use of DT for predicting the onset, treatment (outcome) or development of medical conditions and/or medical procedures. Embodiments may thus be of particular use in relation to medical care management and/or prediction.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 depicts a simplified diagram of training a machine learning algorithm according to an embodiment;
Figure 2 is a flow diagram of a method of training the machine learning algorithm depicted in Figure 2;
Figure 3 is a flow diagram of an alternative method of training a machine learning algorithm according to an embodiment;
Figure 4 depicts a high-level architecture of a generative neural model that may be trained according to a proposed embodiment;
Figure 5 is a simplified schematic diagram of a system according to an embodiment; and
Figure 6 is a simplified schematic diagram of a system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts for training and employing a machine learning algorithm for identifying uncertain DT model parameters. Such concepts may make use of a set of training DTs that is chosen such that it comprises DTs that have achieved a target/predetermined morbidity scenario. The training may then identify uncertain model parameters for the set of DTs. Using the identified uncertain model parameters, the (set of) target DTs may then be run so as to assess whether the target morbidity scenario is achieved.

That is, embodiments propose the training and use of a machine learning algorithm to facilitate the simulation of a morbidity scenario complication (e.g. in advance) with a reduced number of simulation runs/executions.

In the simulation of physical systems, the parameters of the physical model need to be known. However, in many cases, there is uncertainty present on the exact value of such parameters, thus leading to uncertainty in the outcome of the simulation.

To address the uncertain model parameters, a Monte Carlo simulation of the DT model can be performed where many simulations are run for different instantiations (i.e. values) of the model parameters. However, in a medical setting there is only a limited amount of time to perform the simulations and cover relevant morbidity scenarios.

Proposed embodiments seek to address this problem by providing a DT simulation platform where uncertain model parameters are generated by an AI model (such as a generative adversarial neural network). In particular, it is proposed that the generative AI model is trained using a population of DT twins to generate uncertain model parameters for use in simulations such that a target (i.e. predetermined or desired) morbidity scenario is covered with a low/reduced number of runs/executions.

By way of example, it is proposed that a target morbidity scenario may be initially assessed by training a machine learning algorithm using a set of DT models that achieved the target morbidity scenario. In this way, the machine learning algorithm may be trained to generating uncertain model parameters. Subsequently, a set of DT simulations may be run/executed for the generated uncertain model parameters, so as to assess whether the target morbidity scenario is achieved. Use of the uncertain model parameters generated by the machine learning algorithm reduces the number of runs/executions required.

By way of completeness, it is noted that a morbidity scenario is a mode of failure of a medical intervention. A morbidity scenario may thus be defined in terms of the physical state of a DT model. For instance, when certain model variables exceed a predefined value in a simulation, a morbidity scenario may be achieved.

Uncertain Model Parameters are input parameters of a DT model for which some level of uncertainty exists.

A DT population is the collection of DT models for which historical simulation results are available.

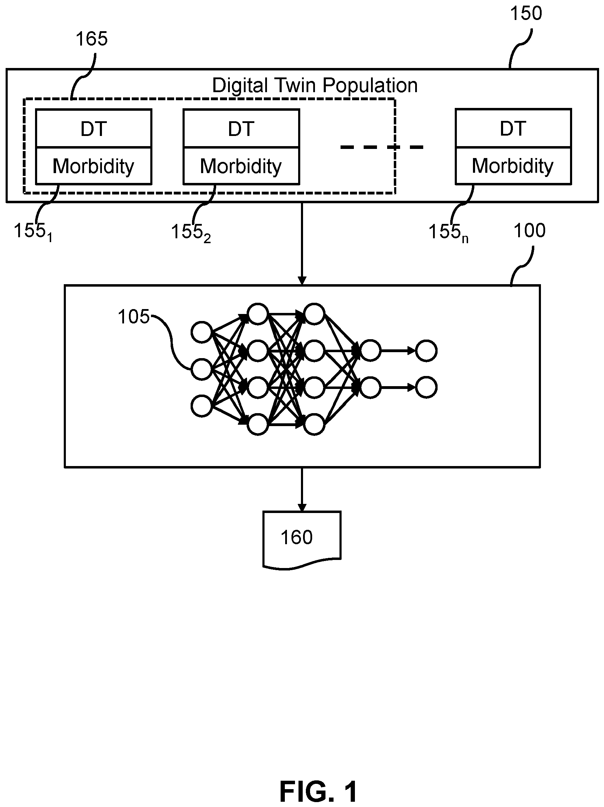

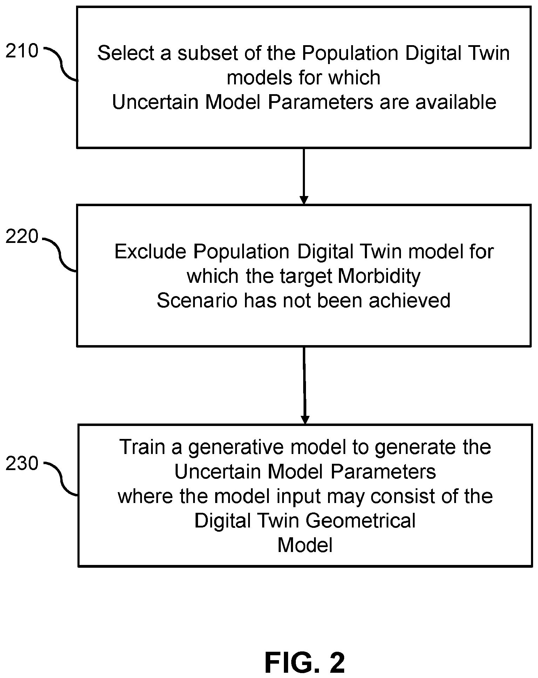

Figures 1 and 2 illustrate an example of training a machine learning algorithm for identifying parameter values for a digital twin of a biological asset of a subject according to an embodiment. The diagrams in the Figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

Referring to Figure 1, there is depicted simplified diagram of training a machine learning algorithm according to an embodiment. Here, the machine-learning algorithm employs an artificial neural network 100, specifically an adversarial neural network.

The structure of an artificial neural network (or, simply, neural network) is inspired by the human brain. Neural networks are comprised of layers, each layer comprising a plurality of neurons 105. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Various approaches to training a machine-learning algorithm are known. Typically, such methods comprise obtaining a training dataset, comprising training input data entries 150 and corresponding training output data entries 160. An initialized machine-learning algorithm is applied to each input data entry 150 to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron 505 may be modified until the error converges. Known methods of modifying a neural network include gradient descent, backpropagation algorithms and so on.

Here, it is proposed for the artificial neural network 100 to generate predicted instances of uncertain model parameters that are likely to achieve a target morbidity scenario. For this purpose, the artificial neural network 100 is trained using a population 150 of digital twin models 155₁, 155₂, ... , 155ₙ.

By way of example, a method of training the machine learning algorithm is illustrated in Figure 2.

In Step 210, a subset 165 of a population 150 of DT models is obtained, wherein each DT of the subset 165 has one or more uncertain model parameters. In step 220, DT models of the subset 165 that did not achieve the target morbidity scenario are excluded (e.g. removed from the population 150) so as to obtain a training set of DTs.

In step 230, the artificial neural network 100 is trained on the resulting training set that consists for each Digital Twin Model of an Uncertain Model Parameter instance, and a Digital Twin Geometrical Model. For a generative neural network, the network is trained to generate instances of Uncertain Model Parameters that are indistinguishable from real instances based on the corresponding Digital Twin Geometrical Model that is input to the network. In other embodiments, the input of the network may consist of other data than a geometrical model. An example is a collection of raw ultrasound measurements taken of a patient without any further processing of the measured ultrasound data.

It will be appreciated that the above-described training method (depicted in Figure 2) may be employed to a generative model based on a population of DT models so as to identify uncertain model parameters.

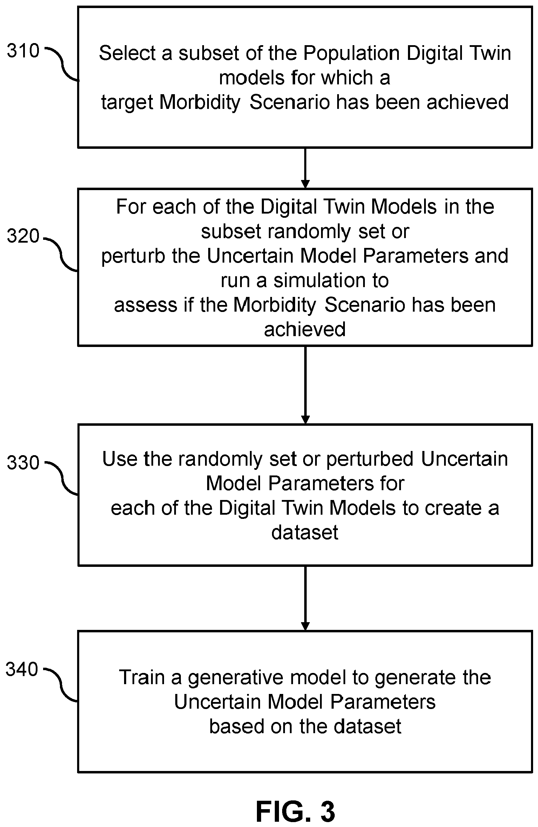

In an alternative embodiment, the method illustrated in Figure 3 may be implemented to train the artificial neural network 100 to generate uncertain DT model parameter instances.

In Step 310, a subset of the population of DT models is selected for which the target Morbidity Scenario has been achieved. For each of the DT models in the subset, the corresponding uncertain model parameters are set randomly or perturbed, and a DT simulation is performed. The resulting morbidity Scenario is then assessed in step 320.

The set of random or perturbed uncertain model parameters may be used to create a dataset with positive and negative examples in Step 330 of the process. The dataset may then be used to train the artificial neural network 100 to generate uncertain model parameters in step 340.

The alternative method of Figure 3 executes additional DT simulations to create a dataset of uncertain model parameter instances that meet the target morbidity scenario.

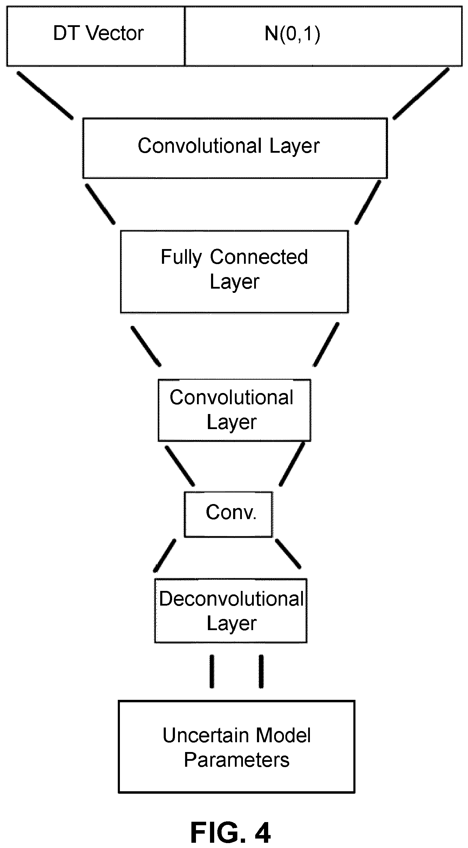

Purely by way of further illustration, an example of a high-level architecture of a generative neural model that may be trained according to one or more proposed concept(s) is shown in Figure 4. The input to the model is a representation of the DT Geometrical Model and a set of random variables distributed according to a known distribution (such as the Gaussian distribution). The neural network model is configured to transform the known probability distribution into the distribution of the uncertain model parameters with knowledge of the DT Geometry and target morbidity scenario. For this purpose, the exemplary model comprises of several layers such as convolution, deconvolution, and fully connected layers. Training may, for example, be accomplished by an adversarial training algorithm. Different generative neural network architectures are known to those with skill in the art and these architectures may be used also to generate uncertain model parameters.

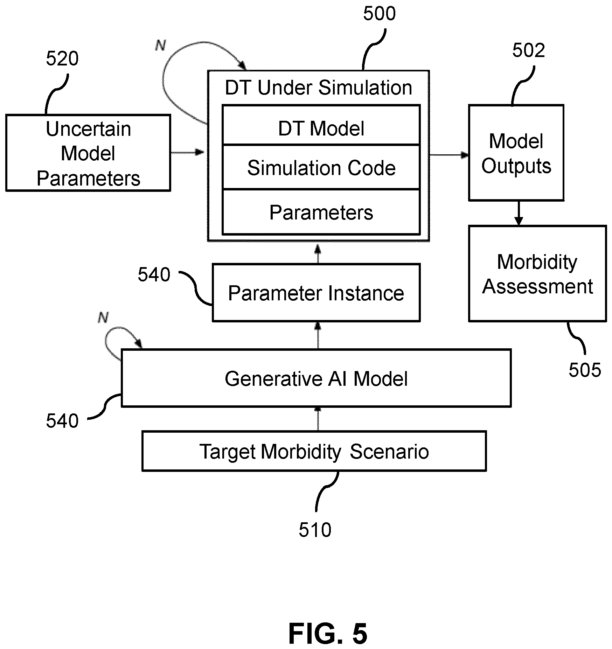

A simplified schematic diagram of a system according to an embodiment is depicted in Figure 5. Specifically, Figure 5 shows a DT model 500 that is under simulation to provide outputs 502 for assessing 505 the morbidity of a treatment or intervention. For this purpose, a target morbidity scenario 510 (i.e. a morbidity scenario of interest) is defined together with a set of relevant uncertain model parameters 520.

For each parameter instance a simulation is run/executed, and the morbidity assessed from the model outputs. The parameter instances 530 are generated by a generative AI model 540 based on the target morbidity scenario 510. The generative AI model 540 is trained on the population of digital twin models for which historical information on intervention and morbidity is available.

From the preceding description, it will be appreciated that there is provided a method for performing an assessment of a target morbidity scenario by DT simulations that comprises the following steps:
Train a generative AI model based on a population of DT models to
generate uncertain model parameter instances that are likely to achieve the target morbidity scenario;
(ii) Sample a set of uncertain model parameter instances from the trained generative AI model;
(iii) Run a DT simulation for each of the uncertain model parameter Instances; and
(iv) Assess whether the target morbidity scenario has been achieved or not for each of the simulations.

Such a method may thus facilitate the simulation of a medical/health complication with a reduced number of simulation runs/executions than conventional DT-based simulation approaches.

Purely by way of further illustration of the proposed concept(s), a proposed embodiment may provide a system to simulate a medical DT model with uncertain model parameters to assess a target morbidity scenario within an allotted simulation. The main components of such a system may be summarized as follows:
(A) Medical digital twin model of organ, organ system, or full patient that is to be simulated;
(B) A Morbidity Scenario of interest defined as a basis to train a generative AI model. The Morbidity Scenario may correspond to a mode of failure of a medical intervention. The generative AI model is trained on a cohort of DT models that has previously achieved the target morbidity scenario in the past and for which at least one uncertain model Parameter is available;
(C) A DT simulation consisting of N runs is performed where each run consists of the following steps: (i) The generative AI model generates an instantiation of the uncertain model parameters for the DT simulation; and (ii) A simulation is run with the instantiation of the uncertain model parameters and an assessment is performed whether the output of the simulation corresponds to the target morbidity scenario.
(D) The results of the N runs are aggregated and presented. Aggregation may consist of grouping the morbidity scenarios into two classes, wherein the classes correspond to simulation runs where the morbidity Scenario has been achieved or not. For each of the classes, the ranges of the uncertain model parameters may be presented. It may be determined which uncertain model parameters contributes/correlates most to the target morbidity scenario. For this purpose, a statistical measure may be used.

Purely by way of yet further illustration of the proposed concept(s), a proposed embodiment may provide a system to train a generative AI model on a population of DTs (for a target (i.e. predefined) morbidity scenario) to generate uncertain model parameters. The main components of such a system may be summarized as follows:
(A) A defined Morbidity Scenario and set of relevant uncertain model parameters.
(B) A training set of DT models that have each previously achieved a target morbidity scenario and each have at least one of the uncertain model parameters available. The elements of the training set may include the geometry of the DT, and basic DT simulation outputs. An example of a basic DT simulation output is a simulated heart ECG for example.
(C) A generative AI model that learns to generate uncertain model parameters based on the geometry of the digital twins contained in the dataset.
   (i) The generative machine learning algorithm may be a generative adversarial neural (GAN) network where the generator is trained to generate uncertain model instances that achieve a target morbidity scenario (a) The input to the generator may be a representation of the DT geometrical model such as a vector of real numbers. The representation may have been obtained by passing a large set of DT Geometries through a dimensionality reduction algorithm such as principle component analysis. (b) A further input to the generator may be a vector of random number drawn from e.g. a Gaussian distribution. Such input will allow the generator to learn a mapping from the distribution to the space of uncertain model parameters.
   (ii) An alternative generative AI model to use is to use a neural importance sampling approach.

By way of illustration, proposed embodiments may be applicable to the following examples:

### Example 1: Optimal stent graft placement in angulated aneurysm necks

Proposed embodiments may be advantageous for endovascular aneurysm repair (EVAR) in subjects with angulated aneurysm necks. EVAR in these subjects may be associated with specific problems such as endoleaks or stent graft migration (e.g. related to proximal stent graft fixation and sealing), with various factors playing a role (as described by van Keulen et al. in a paper entitled "Tips and techniques for optimal stent graft placement in angulated aneurysm necks": van Keulen, JW, Moll, FL and van Herwaarden, JA. 52, 2010, J Vasc Surg, pp. 1081-1086). For procedural planning, computed tomography angiography (CTA) or magnetic resonance angiography (MRA) are used, which allow to assess relevant parameters like length, diameter and angulation of the aneurysm, as well as aortic center lumen line, presence of thrombus, calcification and bulging. It has been previously identified that some of these parameters are difficult to assess leading to uncertainty in the estimated values.

There are various options for a medical professional to influence the outcome of the procedure. For example, prior to EVAR it can be attempted to straighten the aneurysm neck, using a guidewire. In this case, the stiffness of the guidewire influences the chance of successful straightening. Another choice that needs to be made is on the stent graft size, which for straight aneurysm necks are recommended to be 10-20% oversized compared to preoperative neck diameter, which in case of asymmetric placement as may occur in angulated necks could result in effectively much smaller oversizing.

Based on the pre-procedural images, a DT model may be created to simulate stent placements with various guidewire stiffness values and stent sizes, including parameter variation in the other relevant parameters as indicated above. Using a method according to a proposed method, parameter selections could be made to investigate the occurrence of endoleaks and stent graft migration in a limited number of simulation runs. From the results, it may then be seen which (combination of) guide wire stiffness and stent size should be avoided.

### Example 2: Assessing risk of complications

For a medical treatment, potential complications are generally known, such that this knowledge can be used to determine relevant morbidity scenarios to be evaluated. In addition, for most complications, risk factors may be known that help to determine which subjects are at high risk of such complications. However, some complications may be rare, making them difficult to predict. Also, in some cases, it may not just be one or two risk factors/parameters that determine a risk, but the combination of various parameters. In these cases, it may be difficult for a medical professional to assess the risk for an individual patient upfront. Proposed embodiments may help to assess the risk of certain complications for a specific subject.

First, a method according to an embodiment may be employed to generate parameter settings that are used to run DT simulations (based on population DT data chosen such that the target morbidity scenario is more likely to occur than with random sampling). Next, in case the target morbidity scenario did occur in the simulation(s), the probability of the parameter set can be determined using known importance sampling techniques. This additional probability analysis then facilitates a risk assessment. Optionally, this analysis may be done for various treatment options, to be able to select the one with the lowest risk of certain complications.

### Example 3: Evaluate effect of thermal necrosis as a result of bone drilling in orthopaedic procedures

Orthopaedic procedures often require drilling of bone, which, due to heat generation from the friction between the bone and drill bit, can result in death of bone cells. This will, in turn, lead to resorption of bone tissue as an unwanted side effect. Factors that play a role in bone temperature rise, and therefore risk of necrosis, include drilling depth and cortical bone thickness, bone density, drilling speed, drill diameter, axial force, use of graduated versus one-step drilling, and use of irrigation. Numerical simulations can be performed to predict the temperature field in a bone when drilling (as referenced by Mediouni et al. in an paper entitled "An overview of thermal necrosis: present and future": Mediouni, M, Kucklick, T and al, et. 9, 2019, Current Medical Research and Opinion, Vol. 35, pp. 1555-1562). To obtain reliable results, important input parameters for this are, for example, the material properties of each layer of the bone. With these simulations, necrosis can be predicted, as this occurs for temperature above 47°C.

However, although necrosis may lead to detrimental results like loosening of orthopaedic screws, this does not always need to be the case. Bone tissue is continuously remodeling and it is also possible that the necrotic tissue is resorbed and then replaced by healthy bone tissue. Bone remodeling is strongly influenced by mechanical load, and there is a long history of (finite element) bone remodeling studies. Using proposed embodiments, bone necrosis may be predicted in a limited number of simulation runs, after which the severity of side effects of the treatment may be assessed through dedicated finite element modelling taking into account patient geometry and loads.

### Example 4: Selecting the DTs used for generative AI model training based on the individual DT uncertainty

One may consider selection or clustering and weighting of the DT models used for the training of the generative AI model, based on the uncertainty of the individual DTs.

The uncertainty of a DT may be defined based on the size of the confidence interval of its outputs. Alternatively, the uncertainty of a DT can be also based on the duration of the Monte-Carlo simulations run on the DT, for the assessment of the particular morbidity.

It can be expected that training the generative AI model on less "uncertain" DTs will result in narrower search space for the uncertain parameters while running Monte Carlo simulations, and this will help to make morbidity assessment faster.

By including more uncertain DTs, the assessment time may increase. However, this may help for having broader range of the uncertainty parameter assessed.

From the above-described embodiments and examples, it will be understood that the proposed concepts may be particularly relevant to medical facilities (e.g. hospitals) where many users may be involved in a subject care process. In particular, embodiments may be of yet further relevance to academic hospitals.

It is to be understood that the above examples and embodiments are only illustrative and the ordering of one or more of the steps may be modified in alternative embodiments.

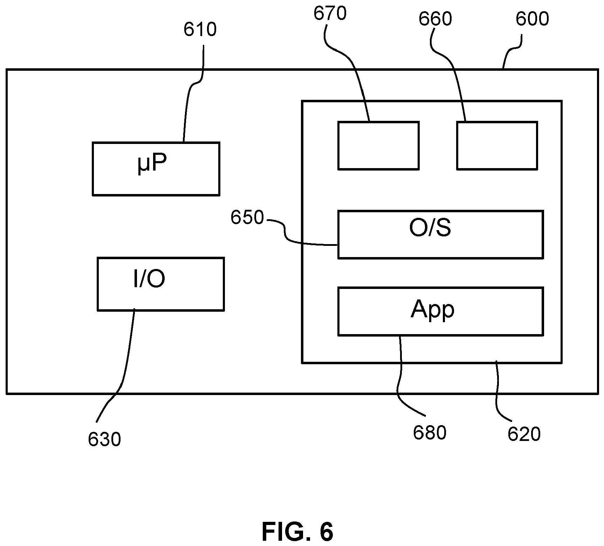

By way of further example, Figure 6 illustrates an example of a computer 600 within which one or more parts of an embodiment may be employed. Various operations discussed above may utilize the capabilities of the computer 600. For example, one or more parts of a system for predicting morbidity scenario risk for a subject may be incorporated in any element, module, application, and/or component discussed herein. In this regard, it is to be understood that system functional blocks can run on a single computer or may be distributed over several computers and locations connected across a cloud-based system (e.g. connected via internet). That is, at least part of the DTs and data may be stored and executed in one or more cloud-based systems, of which the computer 600 may be part.

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 670 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfill the functions of several items recited in the claims.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A method of training a machine learning algorithm for identifying parameter values for a digital twin of a biological asset of a subject, the method comprising:
selecting (210), from a plurality of digital twins (155₁, 155₂, ... , 155ₙ), a subset (165) of the plurality of digital twins, each digital twin of the subset having one or more uncertain model parameters;
removing (220), from the subset, digital twins for which a target morbidity scenario has not been previously obtained, so as to provide a training set of digital twins; and
training (230) the machine learning algorithm based on the remaining digital twins of the subset to generate or identify the uncertain model parameters.

2. The method of claim 1, wherein the training comprises:
defining a value for each of the one or more uncertain model parameters of a first digital twin of the training set;
performing a simulation using the first digital twin to obtain a simulation result; and
adjusting a configuration of the machine learning algorithm based on the simulation result.

3. The method of claim 2, wherein defining a value for each of the one or more uncertain model parameters comprises either: determining a random value; or perturbing an existing parameter value.

4. The method of claim 2 or 3, wherein the training comprises:
determining values for the one or more uncertain model parameters of the first digital twin that provide a simulation result adhering to the target morbidity scenario.

5. The method of claim 2, wherein the training comprises:
adjusting a configuration of the machine learning algorithm so as to decrease a difference between one or more target parameter values of a first digital twin of the training set for obtaining the target morbidity scenario and one or more predicted parameter values that are output by the machine learning component.

6. The method of any of claims 1 to 5, wherein the machine learning algorithm comprises an encoder-type neural network or a generative adversarial neural network.

7. A method for predicting morbidity scenario risk for a subject, the method comprising:
determining parameter values for a digital twin of a biological asset of the subject using a machine learning algorithm trained according to claim 1; and
executing (500) a plurality of simulations with the digital twin for different input values to the digital twin so as obtain a respective plurality of prediction results, each prediction result comprising an indication of morbidity scenario occurrence.

8. The method of claim 7, further comprising:
analyzing the respective plurality of prediction results to identify a digital twin parameter of interest, the digital twin parameter of interest having a level of influence on morbidity scenario occurrence that exceeds a threshold value.

9. The method of claim 8, wherein analyzing comprises:
processing the plurality of prediction results with at least one of: a sampling algorithm and a data clustering algorithm.

10. The method of claim 7, 8 or 9, wherein the machine learning algorithm is trained using a training algorithm configured to receive an array of training inputs and known outputs, wherein the training inputs comprise a representation of a morbidity scenario and the known outputs comprise one or more parameter values for digital twins for predicting the morbidity scenario.

11. The method of any of claims 7 to 10, further comprising communicating at least one of the prediction results to computing device based on the morbidity scenario.

12. A computer program product for training a machine learning algorithm for identifying parameter values for a digital twin of a biological asset of a subject, the computer program product comprising a computer readable storage medium having program instructions embodied therewith, the program instructions executable by a processing unit to cause the processing unit to perform a method comprising:
selecting, from a plurality of digital twins, a subset of the plurality of digital twins, each digital twin of the subset having one or more uncertain model parameters;
removing, from the subset, digital twins for which a target morbidity scenario has not been previously obtained, so as to provide a training set of digital twins; and
training the machine learning algorithm based on the remaining digital twins of the subset to generate or identify the uncertain model parameters.

13. A computer program product for predicting morbidity scenario risk for a subject, the program instructions executable by a processing unit to cause the processing unit to perform a method comprising:
determining parameter values for a digital twin of a biological asset of the subject using a machine learning algorithm trained according to claim 1; and
executing a plurality of simulations with the digital twin for different input values to the digital twin so as obtain a respective plurality of prediction results, each prediction result comprising an indication of morbidity scenario occurrence.

14. A system for training a machine learning algorithm for identifying parameter values for a digital twin of a biological asset of a subject, the system comprising:
a processor arrangement configured to perform the steps of:
selecting, from a plurality of digital twins, a subset of the plurality of digital twins, each digital twin of the subset having one or more uncertain model parameters;
removing, from the subset, digital twins for which a target morbidity scenario has not been previously obtained, so as to provide a training set of digital twins; and
training the machine learning algorithm based on the remaining digital twins of the subset to generate or identify the uncertain model parameters.

15. A system for predicting morbidity scenario risk for a subject, the system comprising:
a processor arrangement configured to perform the steps of:
determining parameter values for a digital twin of a biological asset of the subject using a machine learning algorithm trained according to claim 1; and
executing a plurality of simulations with the digital twin for different input values to the digital twin so as obtain a respective plurality of prediction results, each prediction result comprising an indication of morbidity scenario occurrence.
